# EUROPEAN PATENT SPECIFICATION

(11) **EP 4 446 359 B1**
(45) Date of publication and mention of the grant of the patent: **17.12.2025**
(21) Application number: 24168229.3
(22) Date of filing: 03.04.2024
(51) Int. Cl.: C08G 65/48

(54) **AMINATION PROCESS OF MICROMOLECULAR POLYOXYPROPYLENE ETHER**
AMINIERUNGSVERFAHREN VON MIKROMOLEKULAREM POLYOXYPROPYLENETHER
PROCÉDÉ D'AMINATION D'ÉTHER DE POLYOXYPROPYLÈNE MICROMOLÉCULAIRE

(30) Priority: 10.04.2023 CN 202310369374
(43) Date of publication of application: 16.10.2024
(73) Proprietor: Zhejiang Huangma Technology Co., Ltd., Shaoxing Zhejiang 312000 (CN); Zhejiang Lukean Chemical Co., Ltd., Shaoxing Zhejiang 312000 (CN); Zhejiang Huangma Chemical New Polymer Material Co., Ltd, Shaoxing Zhejiang 312000 (CN); Zhejiang Huangma Shangyi New Material Co., Ltd., Shaoxing Zhejiang 312000 (CN)
(72) Inventor: WANG, Weisong, Shaoxing, 312000 (CN); JIN, Yifeng, Shaoxing, 312000 (CN); YU, Jiang, Shaoxing, 312000 (CN); SONG, Minggui, Shaoxing, 312000 (CN); WANG, Majishi, Shaoxing, 312000 (CN)
(74) Representative: Zaboliene, Reda

(56) References cited:
- CN-A- 106 995 378

## Description

### TECHNICAL FIELD

The present disclosure relates to an amination production method of micromolecular polyoxypropylene ether, belonging to the technical field of preparation or chemical processing of organic compounds.

### BACKGROUND

Amine-terminated polyoxypropylene ether, also called polyether amine (PEA), is a kind of polymer whose main chain has a polyether structure and terminal active functional group is an amine group. Aminated polyether is formed by converting the hydroxyl groups of polyethylene glycol, polypropylene glycol, or ethylene glycol/propylene glycol copolymers into amine groups. By choosing different polyoxyalkyl structures, a series of properties such as reactivity, toughness, viscosity and hydrophilicity of the amino-terminated polyoxypropylene ether can be adjusted, while a terminal amino group provides the possibility for the amino-terminated polyoxypropylene ether to react with various compounds. The special molecular structure of the amino-terminated polyoxypropylene ether endows the amino-terminated polyoxypropylene ether with excellent comprehensive properties. The current commercialized amine-terminated polyoxypropylene ethers include monofunctional, difunctional, and trifunctional series of products with molecular weights ranging from 230 to 5,000. Such compounds can be widely applied to the fields of epoxy resin curing agent, wind energy blade curing agent, polyurethane polyurea elastomer, gasoline cleaning agent, water-based coating, textile finishing agent and epoxy toughening.

At present, the mainstream synthesis method for industrialization is the catalytic amination method, which has the advantages of being stable in product quality and better in compliance with environmental protection requirements when being used for synthesizing amino-terminated polyoxypropylene ether. The catalytic amination method involves an amination reaction between polyether and liquid ammonia under high temperature and pressure in a hydrogen atmosphere for production of the corresponding polyether amine. For polyethers with different structural distributions and molecular weights, the main reaction modes of a hydrogenation reaction include intermittent high-pressure reactor operation and continuous fixed bed operation.

Compared with the continuous fixed bed process, the intermittent high-pressure reactor operation is higher reaction temperature and pressure, and longer in reaction time. Furthermore, an intermittent high-pressure reactor promotes the mixing of reaction raw materials by stirring, but the stirring causes serious damage to a catalyst, which in turn affects the service life of the catalyst. For polyether polyols with an average molecular weight of 600 or less, if a continuous reaction is adopted, the space velocity is low, and the reaction temperature is high. Low space velocity is not conducive to the release of production capacity, and the longer the residence time of low molecular weight polyether in a tubular reactor, the more obvious the reaction cracking and polymerization phenomena. The catalyst surface is prone to carbon deposition, resulting in activity reduction or deactivation of the catalyst.

Based on this, the present disclosure proposes the following application: In view of the advantages of a kettle reaction and a tubular reaction, a two-step method is used to synthesize micromolecular amino-terminated polyoxypropylene ether. Through the reaction in a high-pressure reactor, 70% to 80% of the micromolecular polyoxypropylene ether is terminated within a short period of time, and then the organic amine impurities are removed through deamination and dehydration processes; and then termination is continued through a tubular reactor. Since the amination rate has been completed by 70%-80% in the first step, the second step can increase the space velocity and reduce the contact time of a catalyst. The removal of organic amine impurities in the first step reduces the impact of carbon deposition on the catalyst activity in subsequent reactions. CN 106 995 378 A discloses a method to aminate polypropylene glykol in the presence of ammonia (NH3) and hydrogen using two catalysts while the second catalyst contains Ni, Co and Mo on spinel (MgAl2O4) achieving improved yields and primary amine selectivity.

### SUMMARY

In response to the above-mentioned defects in the processing of the existing amino-terminated micromolecular polyoxypropylene ether, the present application provides an amination process of micromolecular polyoxypropylene ether. To achieve the above objectives, the technical solution adopted in the present application is as follows: an amination process of micromolecular polyoxypropylene ether includes the following steps:

An amination process of micromolecular polyoxypropylene ether includes the following steps:
(1) adding the micromolecular polyoxypropylene ether into a stirring type reaction kettle, introducing liquid ammonia and hydrogen, and implementing terminal hydroxyl amination on the micromolecular polyoxypropylene ether in presence of a catalyst I cat-1 to obtain amine-terminated polyoxypropylene ether with an amination rate being about 70%-80%, where the molar ratio of the liquid ammonia to the micromolecular polyoxypropylene ether is (2-20):1, and after the addition of the micromolecular polyoxypropylene ether and the liquid ammonia, the hydrogen is introduced to allow the pressure in the reaction kettle to reach 1.0-2.0Mpa;
(2) introducing hydrogen, liquid ammonia, and the crude amine-terminated polyoxypropylene ether from the kettle type reaction liquid into a tubular reactor, where the molar ratio of the liquid ammonia to the micromolecular polyoxypropylene ether is (2-20):1, and after the addition of the liquid ammonia and the crude amine-terminated polyoxypropylene ether from the kettle type reaction liquid, the hydrogen is introduced to allow the pressure in the reaction kettle to reach 1.0-2.0Mpa initially; and carrying out a hydroamination reaction in presence of a catalyst II cat-2 to obtain amine-terminated polyoxypropylene ether with an amination rate being about 98% or more.

In step (1), the catalyst I Cat-1 is a copolymer obtained by reduction roasting after a carrier MgAl₂O₄ is loaded with metal salts of Ni, Pt, and La; and in step (2), the catalyst II Cat-2 is composed of a carrier and metal salts loaded thereon, with the carrier selected from γ-Al₂O₃, and the metal salts being Ni, Co, and Mo.

In step (1), the reaction temperature is 90-150°C, the absolute reaction pressure is 3.0-8.0MPa, and the reaction time is 2.0-5.0h.

In step (2), the reaction temperature is 110-170°C, the absolute reaction pressure is 5.0-10.0MPa, and the space velocity is 0.1-0.2g/h/g Cat.

In step (2), the addition molar ratio of the liquid ammonia to the micromolecular polyoxypropylene ether is (5-10):1, and the addition molar ratio of the hydrogen to the micromolecular polyoxypropylene ether is (0.1-2):1.

In step (1), the catalyst I is a carrier MgAl₂O₄ loaded with three metal salts, i.e., Ni(NO₃)₂, Pt(NO₃)₂, and La(NO₃)₃.

In step (1), reduction roasting is performed at 350-500°C during the preparation of the catalyst I.

In step (1), the metal contents in the catalyst I Cat-1 are 0.5-7.0% of Ni, 0.1-0.5% of Pt, 0.4-2.5% of La, 14.0-18.0% of Mg, and 26.0-36.0% of Al.

In step (2), the metal salts in the catalyst II are Ni(NO₃)₂·6H₂O, Co(NO₃)₂·6H₂O, and Mo(NO₃)₃·5H₂O.

In step (2), the metal contents in the catalyst II Cat-2 are 0.5-5.0% of Ni, 0.5-3.0% of Co, 0.2-2.0% of Mo, and 32.0-42.0% of Al.

The structure of the micromolecular polyoxypropylene ether is formed by polymerizing propylene glycol with ethylene oxide and/or propylene oxide, and the molecular weight thereof is 50-600.

The present disclosure is applied to micromolecular amino-terminated polyoxypropylene ether. The polyether is fully contacted with the catalyst through the kettle reaction in the first step, and most of hydroxyl groups are aminated at a lower temperature within a shorter reaction time; the polyether is fully contacted with the catalyst through the reaction in the first step, so that the generation of by-products is reduced, and the space velocity of the subsequent continuous reactions can be effectively improved; and the continuous reaction in the second step not only effectively makes up for the defect of the low amination rate of the kettle reaction, but also prolongs the service life of the kettle catalyst and reduces the process difficulty of the kettle reaction.

### DETAILED DESCRIPTION OF THE EMBODIMENTS

### Preparation method of micromolecular polyoxypropylene ether:

3mol (228g) of propylene glycol was added into a reaction kettle, 3.0g of KOH was then added, and the obtained mixture was heated up to 100-110°C for dehydration for 30min. The product was heated again and introduced with 9mol (540g) of propylene oxide slowly. During the introduction of propylene oxide, the temperature was kept within 115-125°C. After the addition of propylene oxide was finished, the reaction was continued for 30min before discharging. The initial product polyether was absorbed by an adsorbent, and then micromolecular polyether with a molecular weight of 240 was obtained.

### Example 1. Preparation of catalyst CAT-1

A catalyst was prepared according to an impregnation method. Metals Pt, Ni, and La in amounts accounting for 0.25%, 3.5%, and 1.2% of the total weight of the catalyst were prepared into an aqueous solution with a certain concentration of three metal salts, i.e., Ni(NO₃)₂, Pt(NO₃)₂, and La(NO₃)₃. Impregnation was performed for 5h at 70°C until MgAl₂O₄ particles accounting for 95% of the total amount of the catalyst were formed on a spherical carrier. After being dried, the catalyst was subjected to reduction roasting at 420°C, so that a catalyst CAT-1 was obtained.

### Example 2. Preparation of catalyst CAT-2

Metal salts, i.e., Ni(NO₃)₂·6H₂O, Co(NO₃)₂·6H₂O and Mo(NO₃)₃·5H₂O were dissolved into a solution at a certain temperature according to a certain proportion: the metal contents were 2.5% of Ni, 2.0% of Co, and 1.0% of Mo. 300g of a γ-Al₂O₃ carrier was impregnated in batches for 50min, and then the water was evaporated to dryness under the conditions that the water temperature was 70°C and the vacuum degree was -0.080 to -0.095MPa. After that, the evaporated processed material was placed in a vacuum oven at 120°C for dehydration for about 3h to remove free water and crystalline water. After drying, the product was placed in a muffle furnace for roasting. The roasting was performed at 450°C for 3h, the product was then put into a tubular high-temperature furnace for reduction under hydrogen atmosphere, the reduction was performed at the temperature of about 420°C for 3h, and finally, a catalyst II (Cat-2) was obtained, where the metal contents in the γ-Al₂O₃ were 2.5% of Ni, 2.0% of Co, and 1.0% of Mo.

### Example 3. Catalyst use in kettle reaction

The catalysts of Examples 1 and 2 were selected respectively, and micromolecular polyoxypropylene ether was prepared by a kettle reactor. The amination reaction temperature was 120°C, the absolute reaction pressure was 7.0MPa, and the molar ratio of liquid ammonia to the micromolecular polyoxypropylene ether was 10:1, that was, 340g of the liquid ammonia and 460g of the polyoxypropylene ether 230 were available in the reaction. After the addition of the micromolecular polyoxypropylene ether and the liquid ammonia was finished, hydrogen was introduced to make the reactor pressure reach 2.0MPa. The reaction lasted for 4h. The adaptability of the catalysts loaded on two kinds of carriers to the kettle reaction was investigated.

**Table 1 Performance comparison of catalysts loaded on different carriers**

| Type of catalyst | Reaction pressure (MPa) | Service life (times) | Average amination rate % | Primary amine selectivity % | Average crushing rate (%) |
|---|---|---|---|---|---|
| Catalyst of Example 2 | 7.0 | 24 | 97.7 | 97.5 | 0.26 |
| Catalyst of Example 1 | 7.0 | 45 | 98.9 | 99.0 | 0.11 |

Table 1 shows that the MgAl2O4 carrier is loaded with metal elements such as Pt, Ni, and La in the kettle reaction, the low crushing rate and the long service life indicate that the catalyst is very suitable for the kettle amination reaction, and the average amination rate and the selectivity indicate that the catalyst (CAT-1) has higher activity.

### Example 4

A catalyst CAT-1 was added to a high-pressure reactor, micromolecular polyoxypropylene ether 230(460g, 2mol) was added, liquid ammonia (170g, 10mol) was then added, hydrogen was introduced until the initial pressure of the high-pressure reactor was 1.5MPa, and the reaction temperature and the reaction pressure were respectively controller to be was 130°C and 7.0MPa. Under these reaction conditions, the effects of different reaction time on the amination rate and selectivity were investigated. The calculation method was: amination rate=total amine value/hydroxyl value of micromolecular polyoxypropylene ether*100%; amination selectivity=primary amine value/total amine value*100%. The determination method for total amine value and tertiary amine value was HMJC/ZD(J)-027-2023. The determination method for primary amine content was HMJC/ZD(J)-037-2023. The determination method for hydroxyl value was GB/T 7383.

**Table 2 Test results of kettle reactor**

| Reaction time (h) | Amination rate % | Primary amine selectivity % |
|---|---|---|
| 1 | 42.0 | 100 |
| 2 | 72.0 | 99.7 |
| 3 | 83.0 | 99.6 |
| 10 | 89.0 | 99.5 |
| 12 | 94.5 | 99.4 |
| 15 | 97.0 | 99.4 |

As can be seen from Table 2, the amination rate (less than 80%) of the kettle reaction has a fast reaction rate, which can usually be achieved within 3-5h. However, to further increase the amination rate, it requires several times longer reaction time, which is not conducive to the service life of the catalyst and the increase in production capacity.

### Example 5

A catalyst CAT-2 was loaded into a tubular reactor for reaction, and polyoxypropylene ether (230) was selected to react with liquid ammonia and hydrogen for amination at a reaction temperature of 150°C and a reaction pressure of 8.0MPa. The molar ratio of the liquid ammonia to the micromolecular polyoxypropylene ether was 7:1, and the molar ratio of the hydrogen to the micromolecular polyoxypropylene ether was 1:1. The amination rate and selectivity of different treated micromolecular polyoxypropylene ethers in the reaction system were compared.

**Table 3 Test results of tubular reactor**

| Polyoxypropylene ether | Organic amine impurities % | Feed space velocity g/h/g/Cat | Amination rate % | Selectivity | Lifetime (h) |
|---|---|---|---|---|---|
| Unaminated | 0 | 0.05 | 85.5 | 99.8 | / |
| Aminated by 50% | 0 | 0.1 | 95.8 | 99.6 | / |
| Aminated by 70% | 1.0 | 0.1 | 99.2 | 99.2 | 2400 |
| Aminated by 70% | 0 | 0.15 | 99.8 | 99.8 | 3100 |
| Aminated by 80% | 1.5 | 0.1 | 99.0 | 93.2 | / |
| Aminated by 80% | 0 | 0.15 | 99.8 | 99.5 | / |

It can be seen from Example 5 that the intermediate amination rate of the product subjected to amination in the reaction kettle of the first step is 70-80%, which can greatly improve the space velocity; and the organic amine impurities have a great influence on the selectivity of the product, and further evidence has shown that the impurities have a significant impact on the lifetime of the catalyst CAT-2.

The present disclosure has been described in detail above in conjunction with specific embodiments and exemplary examples, but these descriptions should not be construed as limiting the present disclosure. protection scope of the present disclosure shall be subject to the appended claims.

## Claims

1. An amination process of micromolecular polyoxypropylene ether, comprising the following steps:
(1) adding the micromolecular polyoxypropylene ether into a stirring type reaction kettle, introducing liquid ammonia and hydrogen, and implementing terminal hydroxyl amination on the micromolecular polyoxypropylene ether in presence of a catalyst I cat-1 to obtain amine-terminated polyoxypropylene ether with an amination rate being about 70%-80%, wherein the molar ratio of the liquid ammonia to the micromolecular polyoxypropylene ether is (2-20):1, and after the addition of the micromolecular polyoxypropylene ether and the liquid ammonia, the hydrogen is introduced to allow the pressure in the reaction kettle to reach 1.0-2.0Mpa;
(2) introducing hydrogen, liquid ammonia, and the crude amine-terminated polyoxypropylene ether from the kettle type reaction liquid into a tubular reactor, wherein the molar ratio of the liquid ammonia to the micromolecular polyoxypropylene ether is (2-20):1, and after the addition of the liquid ammonia and the crude amine-terminated polyoxypropylene ether from the kettle type reaction liquid, the hydrogen is introduced to allow the pressure in the reaction kettle to reach 1.0-2.0Mpa initially; and carrying out a hydroamination reaction in presence of a catalyst II cat-2 to obtain amine-terminated polyoxypropylene ether with an amination rate being about 98% or more;
in step (1), the catalyst I Cat-1 being a copolymer obtained by reduction roasting after a carrier MgAl₂O₄ is loaded with metal salts of Ni, Pt, and La; and in step (2), the catalyst II Cat-2 being composed of a carrier and metal salts loaded thereon, with the carrier selected from γ-Al₂O₃, and the metal salts being Ni, Co, and Mo.

2. The amination process of the micromolecular polyoxypropylene ether according to claim 1, wherein in step (1), the reaction temperature is 90-150°C, the absolute reaction pressure is 3.0-8.0MPa, and the reaction time is 2.0-5.0h.

3. The amination process of the micromolecular polyoxypropylene ether according to claim 1, wherein in step (2), the reaction temperature is 110-170°C, the absolute reaction pressure is 5.0-10.0MPa, and the space velocity is 0.1-0.2g/h/g Cat.

4. The amination process of the micromolecular polyoxypropylene ether according to claim 1, wherein in step (2), the addition molar ratio of the liquid ammonia to the micromolecular polyoxypropylene ether is (5-10):1, and the addition molar ratio of the hydrogen to the micromolecular polyoxypropylene ether is (0.1-2):1.

5. The amination process of the micromolecular polyoxypropylene ether according to claim 1, wherein in step (1), the catalyst I is a carrier MgAl₂O₄ loaded with three metal salts, i.e., Ni(NO₃)₂, Pt(NO₃)₂, and La(NO₃)₃.

6. The amination process of the micromolecular polyoxypropylene ether according to claim 5, wherein in step (1), reduction roasting is performed at 350-500°C during the preparation of the catalyst I.

7. The amination process of the micromolecular polyoxypropylene ether according to claim 5, wherein in step (1), the metal contents in the catalyst I Cat-1 are 0.5-7.0% of Ni, 0.1-0.5% of Pt, 0.4-2.5% of La, 14.0-18.0% of Mg, and 26.0-36.0% of Al.

8. The amination process of the micromolecular polyoxypropylene ether according to claim 1, wherein in step (2), the metal salts in the catalyst II are Ni(NO₃)₂·6H₂O, Co(NO₃)₂·6H₂O, and Mo(NO₃)₃·5H₂O.

9. The amination process of the micromolecular polyoxypropylene ether according to claim 8, wherein in step (2), the metal contents in the catalyst II Cat-2 are 0.5-5.0% of Ni, 0.5-3.0% of Co, 0.2-2.0% of Mo, and 32.0-42.0% of Al.

10. The amination process of the micromolecular polyoxypropylene ether according to claim 1, wherein the structure of the micromolecular polyoxypropylene ether is formed by polymerizing propylene glycol with ethylene oxide and/or propylene oxide, and the molecular weight thereof is 50-600.

## Patentansprüche

1. Aminierungsverfahren von mikromolekularem Polyoxypropylenether, das die folgenden Schritte umfasst:
(1) Zugeben des mikromolekularen Polyoxypropylenethers in einen rührenden Reaktionskessel, Einleiten von flüssigem Ammoniak und Wasserstoff und Durchführen einer terminalen Hydroxylaminierung des mikromolekularen Polyoxypropylenethers in Gegenwart eines Katalysators I Cat-1, um aminterminierten Polyoxypropylenether mit einer Aminierungsrate von etwa 70 %-80 % zu erhalten, wobei das Molverhältnis des flüssigen Ammoniaks zum mikromolekularen Polyoxypropylenether (2-20):1 beträgt, und nach dem Zugeben des mikromolekularen Polyoxypropylenethers und des flüssigen Ammoniaks der Wasserstoff eingeleitet wird, um den Druck im Reaktionskessel auf 1,0-2,0 MPa zu bringen;
(2) Einleiten von Wasserstoff, flüssigem Ammoniak und dem rohen aminterminierten Polyoxypropylenether aus der Kesselreaktionsflüssigkeit in einen Rohrreaktor, wobei das Molverhältnis des flüssigen Ammoniaks zum mikromolekularen Polyoxypropylenether (2-20):1 beträgt, und nach dem Zugeben des flüssigen Ammoniaks und des rohen aminterminierten Polyoxypropylenethers aus der Kesselreaktionsflüssigkeit der Wasserstoff eingeleitet wird, um den Druck im Reaktorkessel anfänglich auf 1,0-2,0 MPa zu bringen; und Durchführen einer Hydroaminierungsreaktion in Gegenwart eines Katalysators II Cat-2, um aminterminierten Polyoxypropylenether mit einer Aminierungsrate von etwa 98 % oder mehr zu erhalten;
wobei in Schritt (1) der Katalysator I Cat-1 ein Copolymer ist, das durch Reduktionsröstung erhalten wird, nachdem ein Träger MgAl₂O₄ mit Metallsalzen von Ni, Pt und La beladen wurde; und in Schritt (2) der Katalysator II Cat-2 aus einem Träger und darauf beladenen Metallsalzen zusammengesetzt ist, wobei der Träger aus γ-Al₂O₃ ausgewählt ist und die Metallsalze Ni, Co und Mo sind.

2. Aminierungsverfahren von mikromolekularem Polyoxypropylenether nach Anspruch 1, wobei in Schritt (1) die Reaktionstemperatur 90-150 °C beträgt, der absolute Reaktionsdruck 3,0-8,0 MPa beträgt und die Reaktionszeit 2,0-5,0 h beträgt.

3. Aminierungsverfahren von mikromolekularem Polyoxypropylenether nach Anspruch 1, wobei in Schritt (2) die Reaktionstemperatur 110 bis 170 °C beträgt, der absolute Reaktionsdruck 5,0 bis 10,0 MPa beträgt und die Raumgeschwindigkeit 0,1 bis 0,2 g/h/g Cat beträgt.

4. Aminierungsverfahren von mikromolekularem Polyoxypropylenether nach Anspruch 1, wobei in Schritt (2) das Zugebe-Molverhältnis des flüssigen Ammoniaks zum mikromolekularen Polyoxypropylenether (5-10):1 beträgt und das Zugebe-Molverhältnis des Wasserstoffs zum mikromolekularen Polyoxypropylenether (0,1-2):1 beträgt.

5. Aminierungsverfahren von mikromolekularem Polyoxypropylenether nach Anspruch 1, wobei in Schritt (1) der Katalysator I ein Träger MgAl₂O₄ ist, der mit drei Metallsalzen, nämlich Ni(NO₃)₂, Pt(NO₃)₂ und La(NO₃)₃, beladen ist.

6. Aminierungsverfahren von mikromolekularem Polyoxypropylenether nach Anspruch 5, wobei in Schritt (1) während der Herstellung des Katalysators I die Reduktionsröstung bei 350-500 °C durchgeführt wird.

7. Aminierungsverfahren von mikromolekularem Polyoxypropylenether nach Anspruch 5, wobei in Schritt (1) die Metallgehalte in dem Katalysator I Cat-1 0,5-7,0 % Ni, 0,1-0,5 % Pt, 0,4-2,5 % La, 14,0-18,0 % Mg und 26,0-36,0 % Al betragen.

8. Aminierungsverfahren von mikromolekularem Polyoxypropylenether nach Anspruch 1, wobei in Schritt (2) die Metallsalze in dem Katalysator II Ni(NO₃)₂·6H₂O, Co(NO₃)₂·6H₂O und Mo(NO₃)₃·5H₂O sind.

9. Aminierungsverfahren von mikromolekularem Polyoxypropylenether nach Anspruch 8, wobei in Schritt (2) die Metallgehalte in dem Katalysator II Cat-2 0,5-5,0 % Ni, 0,5-3,0 % Co, 0,2-2,0 % Mo und 32,0-42,0 % Al betragen.

10. Aminierungsverfahren von mikromolekularem Polyoxypropylenether nach Anspruch 1, wobei die Struktur des mikromolekularen Polyoxypropylenethers durch Polymerisieren von Propylenglykol mit Ethylenoxid und/oder Propylenoxid gebildet ist und das Molekulargewicht davon 50-600 beträgt.

## Revendications

1. Procédé d'amination de l'éther de polyoxypropylène micromoléculaire, comprenant les étapes suivantes consistant à :
(1) ajouter l'éther de polyoxypropylène micromoléculaire dans un réacteur à agitation, introduire de l'ammoniac liquide et de l'hydrogène, et réaliser une amination hydroxyle terminale sur l'éther de polyoxypropylène micromoléculaire en présence d'un catalyseur I cat-1 afin d'obtenir un éther de polyoxypropylène à terminaison amine avec un taux d'amination qui est d'environ 70 % à 80 %, dans lequel le rapport molaire entre l'ammoniac liquide et l'éther de polyoxypropylène micromoléculaire est de (2 à 20):1, et après l'ajout de l'éther de polyoxypropylène micromoléculaire et de l'ammoniac liquide, l'hydrogène est introduit afin de permettre à la pression dans le réacteur d'atteindre 1,0 à 2,0 Mpa ;
(2) introduire de l'hydrogène, de l'ammoniac liquide et l'éther de polyoxypropylène brut à terminaison amine à partir du liquide de réaction de type cuve dans un réacteur tubulaire, dans lequel le rapport molaire entre l'ammoniac liquide et l'éther de polyoxypropylène micromoléculaire est de (2 à 20):1, et après l'ajout de l'ammoniac liquide et de l'éther de polyoxypropylène brut à terminaison amine à partir du liquide de réaction de type cuve, l'hydrogène est introduit afin de permettre à la pression dans le réacteur d'atteindre 1,0 à 2,0 MPa initialement ; et réaliser une réaction d'hydroamination en présence d'un catalyseur Il cat-2 afin d'obtenir un éther de polyoxypropylène à terminaison amine avec un taux d'amination qui est d'environ 98 % ou plus ;
à l'étape (1), le catalyseur I Cat-1 étant un copolymère obtenu par grillage réducteur après qu'un support MgAl₂O₄ ait été chargé de sels métalliques de Ni, Pt et La ; et à l'étape (2), le catalyseur II Cat-2 étant composé d'un support et de sels métalliques chargés sur celui-ci, le support étant choisi parmi γ-Al₂O₃, et les sels métalliques étant Ni, Co et Mo.

2. Procédé d'amination de l'éther de polyoxypropylène micromoléculaire selon la revendication 1, dans lequel, à l'étape (1), la température de réaction est comprise entre 90 et 150 °C, la pression de réaction absolue est comprise entre 3,0 et 8,0 MPa, et la durée de réaction est comprise entre 2,0 et 5,0 h.

3. Procédé d'amination de l'éther de polyoxypropylène micromoléculaire selon la revendication 1, dans lequel, à l'étape (2), la température de réaction est comprise entre 110 et 170 °C, la pression de réaction absolue est comprise entre 5,0 et 10,0 MPa, et la vitesse spatiale est comprise entre 0,1 et 0,2 g/h/g Cat.

4. Procédé d'amination de l'éther de polyoxypropylène micromoléculaire selon la revendication 1, dans lequel, à l'étape (2), le rapport molaire d'addition entre l'ammoniac liquide et l'éther de polyoxypropylène micromoléculaire est de (5 à 10):1, et le rapport molaire d'addition entre l'hydrogène et l'éther de polyoxypropylène micromoléculaire est de (0,1 à 2):1.

5. Procédé d'amination de l'éther de polyoxypropylène micromoléculaire selon la revendication 1, dans lequel, à l'étape (1), le catalyseur I est un support MgAl₂O₄ chargé de trois sels métalliques, à savoir Ni(NO₃)₂, Pt(NO₃)₂ et La(NO₃)₃.

6. Procédé d'amination de l'éther de polyoxypropylène micromoléculaire selon la revendication 5, dans lequel, à l'étape (1), un grillage réducteur est effectué à 350 à 500 °C pendant la préparation du catalyseur I.

7. Procédé d'amination de l'éther de polyoxypropylène micromoléculaire selon la revendication 5, dans lequel, à l'étape (1), les teneurs en métaux dans le catalyseur I Cat-1 sont les suivantes : 0,5 à 7,0 % de Ni, 0,1 à 0,5 % de Pt, 0,4 à 2,5 % de La, 14,0 à 18,0 % de Mg, et 26,0 à 36,0 % d'Al.

8. Procédé d'amination de l'éther de polyoxypropylène micromoléculaire selon la revendication 1, dans lequel, à l'étape (2), les sels métalliques dans le catalyseur II sont Ni(NO₃)₂·6H₂O, Co(NO₃)₂·6H₂O, and Mo(NO₃)₃·5H₂O.

9. Procédé d'amination de l'éther de polyoxypropylène micromoléculaire selon la revendication 8, dans lequel, à l'étape (2), les teneurs en métaux dans le catalyseur Il Cat-2 sont les suivantes : 0,5 à 5,0 % de Ni, 0,5 à 3,0 % de Co, 0,2 à 2,0 % de Mo, et 32,0 à 42,0 % d'AI.

10. Procédé d'amination de l'éther de polyoxypropylène micromoléculaire selon la revendication 1, dans lequel la structure de l'éther de polyoxypropylène micromoléculaire est formée par polymérisation de propylène glycol avec de l'oxyde d'éthylène et/ou de l'oxyde de propylène, et le poids moléculaire de celui-ci est compris entre 50 et 600.
